# FASCICULE DE BREVET EUROPEEN

(11) **EP 0 573 334 B1**
(45) Date de publication et mention de la délivrance du brevet: **19.01.2000**
(21) Numéro de dépôt: 93401363.2
(22) Date de dépôt: 28.05.1993
(51) Int. Cl.: A61N 1/05

(54) **Sonde cardiaque extractible**
Ausziehbare Herzsonde
Extractable cardiac catheter

(30) Priorité: 03.06.1992 FR 9206704
(43) Date de publication de la demande: 08.12.1993
(73) Titulaire: ELA MEDICAL (Société anonyme), F-92541 Montrouge Cédex (FR)
(72) Inventeur: Brehier, Jacques, F-7200 Le Mans (FR)
(74) Mandataire: Laget, Jean-Loup

(56) Documents cités:
- EP-A- 0 041 254

## Description

L'invention est relative à une sonde cardiaque extractible et son procédé de mise en oeuvre.

Les sondes cardiaques sont utilisées pour la stimulation cardiaque et pour recueillir des signaux électriques en provenance du coeur. Une sonde comporte deux extrémités : l'une appelée proximale, qui est, dans le cadre de la stimulation cardiaque, destinée à être connectée au générateur de signaux, l'autre appelée distale et qui est destinée à être mise en contact avec la paroi interne du muscle cardiaque.

La sonde comporte un conducteur électrique constitué par un fil métallique enroulé en spirale et disposé à l'intérieur d'une gaine souple biocompatible, par exemple en caoutchouc au silicone. Le conducteur est relié à une électrode de forme annulaire ou cylindrique disposée à l'extrémité distale de la sonde.

Divers incidents peuvent surgir lors de l'utilisation de telles sondes :
- d'une part, il est nécessaire de remplacer une sonde ne fonctionnant pas correctement par une sonde neuve,
- d'autre part, des foyers d'infection peuvent se déclarer à l'extrémité ou le long de la sonde.

L'extraction de la sonde est impérative dans les deux cas précités : à cet effet, un procédé connu d'extraction consiste à tirer sur la sonde en appliquant une traction constante de l'ordre de 1N à 5N jusqu'à détachement de la sonde de la paroi du muscle cardiaque ; cette opération longue et pénible pour le patient dure souvent plusieurs jours.

Les documents DE 3 937 594, US 4 574 800 et US 4 988 347 décrivent trois dispositifs différents d'extraction de sonde cardiaque, dont le point commun est le suivant :
ces dispositifs expansibles par augmentation de leur diamètre extérieur sont insérés à l'intérieur du conducteur électrique enroulé en spirale jusqu'au voisinage de l'extrémité distale de la sonde ; puis sont fixés par expansion de leur diamètre extérieur à cette extrémité distale de manière à pouvoir exercer une traction sur cette extrémité distale.

L'inconvénient résultant de l'emploi de ces dispositifs connus est le risque d'arrachage de la fibrine entourant l'extrémité distale de la sonde et, éventuellement, d'une partie du myocarde.

L'invention a pour but de remédier aux inconvénients des dispositifs et procédés connus, en proposant une sonde cardiaque extractible, dont la mise en oeuvre réduit fortement les risques d'arrachage de fibrine ou d'une partie du myocarde.

Le document EP-A-41 254 décrit une sonde cardiaque implantable présentant à son extrémité un manchon portant des moyens de fixation à la paroi cardiaque. Dans cette sonde, le cathéter et l'électrode peuvent être désolidarisés du manchon, par un effort axial, avec l'aide d'un outil tubulaire glissant sur le cathéter et venant en appui sur le manchon.

L'invention a pour but de réduire l'effort axial nécessaire pour désolidariser le cathéter et le manchon de fixation.

L'invention a pour objet une sonde cardiaque extractible au moyen d'un outil, notamment pour stimulateur cardiaque, du type comportant: un cathéter à gaine souple portant une électrode à son extrémité distale destinée à être placée contre une paroi interne du coeur; et un manchon sensiblement cylindrique portant des moyens de fixation de la sonde sur la paroi cardiaque, apte à être solidarisé au cathéter ; ledit cathéter pouvant être désolidarisé dudit manchon par action dudit outil pour autoriser le retrait du cathéter et le maintien en place du manchon sur la paroi cardiaque ; le manchon et le cathéter comportant des formes coopérantes pour assurer leur solidarisation, caractérisée en ce que :
- le manchon comporte, du côté tourné vers l'extrémité proximale de la sonde, une partie sensiblement tronconique adaptée à recevoir l'outil d'écartement tubulaire et,
- le manchon est au moins partiellement déformable par agrandissement du diamètre de son passage intérieur au moyen de l'outil d'écartement tubulaire, pour permettre l'extraction du cathéter par coulissement.

Selon d'autres modes de réalisation de l'invention :
- le manchon et le cathéter comportent des formes coopérantes, telles que des gorges coopérant avec des anneaux de retenue ou des bossages coopérant avec des cavités ;
- le manchon comporte à son extrémité distale une pièce sensiblement indéformable ;
- ladite pièce indéformable est un anneau de retenue en matériau radio-opaque ;
- le cathéter comporte, au voisinage de son extrémité distale, un insert de retenue fixé sur la gaine souple;
- ledit insert est un tube en matériau radio-opaque.

Un procédé de mise en oeuvre d'une sonde extractible selon l'invention peut comporter les étapes suivantes :
- placer autour du cathéter de la sonde un écarteur et le faire coulisser de l'extrémité proximale vers l'extrémité distale de la sonde ;
- introduire l'écarteur entre le manchon et le cathéter pour déformer élastiquement le manchon et accroître le diamètre de passage intérieur du manchon ;
- retirer le cathéter par traction à travers le manchon et l'écarteur ;
   Le procédé peut comporter en outre les étapes suivantes :
- laisser en place le manchon après le retrait du cathéter ;
- maintenir le manchon grâce à l'écarteur, de manière à conserver le diamètre de passage correspondant au diamètre extérieur du cathéter ;
- insérer un cathéter neuf à travers l'écarteur et le manchon jusqu'à la paroi du myocarde,
- retirer l'écarteur en laissant en place le cathéter rendu solidaire du manchon après retour élastique du manchon sur le cathéter ;
   L'invention sera mieux comprise grâce à la description qui va suivre donnée à titre d'exemple non limitatif en référence aux dessins annexés dans lesquels :
   - La figure 1 représente une vue en coupe schématique d'une sonde extractible selon l'invention fixée sur le myocarde en position d'utilisation.
   - La figure 2 représente une vue en coupe schématique d'une sonde extractible selon l'invention dont l'électrode est en cours d'extraction.
   - La figure 3 représente une vue en coupe schématique d'un manchon de sonde selon l'invention en position élargie.

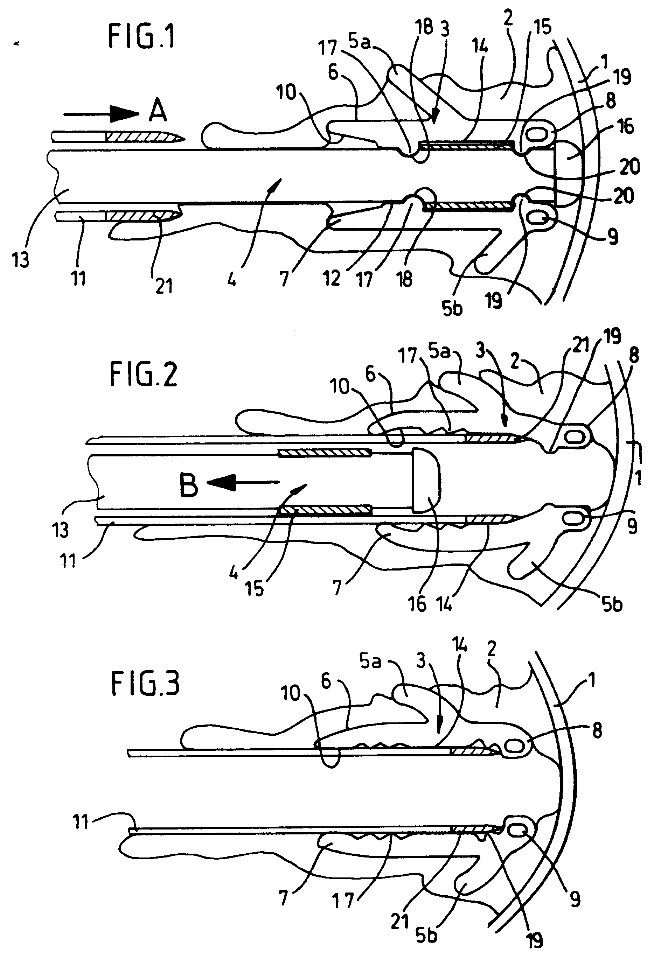

En référence à la figure 1, on a implanté sur la paroi 1 du muscle cardiaque une sonde selon l'invention, dont l'extrémité distale est entourée de fibrine 2.

La sonde selon l'invention se compose de deux parties : un manchon 3 d'implantation et de retenue, et un cathéter 4.

Le manchon 3 extérieur au cathéter 4 comporte des barbules flexibles de retenue 5a, 5b, en saillie extérieure par rapport à la paroi extérieure 6 du manchon sensiblement cylindrique, en contact avec la fibrine 2.

Le manchon 3 déformable à son extrémité proximale 7 est renforcé à son extrémité distale 8 par une pièce 9 sensiblement indéformable : la pièce 9 est de préférence en forme d'anneau constitué de métal ou d'un matériau radio-opaque, par exemple du platine-iridium.

L'extrémité déformable 7 présente intérieurement une première partie sensiblement tronconique 10 tournée vers l'extrémité proximale de la sonde de manière à permettre l'introduction d'un écarteur tubulaire 11 progressant le long de la sonde pour en décoller la fibrine, selon le sens de la flèche A.

La partie tronconique 10 se prolonge continûment par une partie sensiblement cylindrique 12 de diamètre correspondant au diamètre de la gaine souple 13 du cathéter, laquelle se prolonge par une autre partie sensiblement cylindrique 14 de diamètre supérieur adapté à un insert 15 ou autre ergot de préférence en matériau radio-opaque.

L'insert 15 solidaire de la gaine souple 13 est avantageusement constitué par un tube métallique enserrant la gaine souple 13 au voisinage de l'électrode 16 du cathéter 4.

Selon l'invention, la sonde comporte un manchon 3 et un cathéter 4 aptes à être solidarisés entre eux avant l'implantation, et à rester solidaires en position de stimulation cardiaque au moyen de formes coopérantes 17, 18 ; 19, 20 élastiquement déformables au moins du côté du manchon.

La solidarisation précitée du cathéter 4 à l'intérieur du manchon 3 ancré dans la fibrine 2 au moyen des barbules 5a, 5b réalise ainsi l'ancrage de la sonde dans la fibrine 2, la fixation sur le muscle cardiaque 1, et permet la stimulation de celui-ci.

Les formes coopérantes 17, 18 ; 19, 20 par exemple constituées en creux sur la gaine 13 et en relief intérieur sur le manchon 3 sont de préférence directement venues de matière lors de la fabrication du manchon 3 et de la gaine 13, en matériau biocompatible, par exemple du type caoutchouc au silicone.

Les formes coopérantes 17, 18 ; 19, 20 sont par exemple constituées par des anneaux en saillie intérieure et des gorges de retenue correspondantes ; mais elles peuvent également, en variante, être constituées de bossages en relief 17, 19 et de cavités 18, 20 adaptées auxdits bossages. Il est également possible de ne prévoir les formes en relief 17, 19 que sur le manchon, la gaine souple du cathéter se déformant élastiquement pour épouser ces formes.

L'essentiel est que l'insertion d'un outil tubulaire 11 d'écartement décolle la fibrine et déforme le manchon 3 suffisamment en agrandissant son diamètre de passage intérieur pour désolidariser les formes coopérantes 17, 18 ; 19, 20 et permettre l'extraction du cathéter 4 par un effort modéré correspondant au décollement de l'électrode 16 de la paroi 1 du myocarde.

En référence à la figure 2, on a effectué l'insertion de l'écarteur 11 au moins jusqu'au niveau de la partie cylindrique 14 du manchon 3.

L'écarteur 11, avantageusement constitué par une gaine cylindrique à extrémité effilée et radio-opaque 21, présente un diamètre intérieur correspondant au coulissement avec jeu de la gaine 13, de l'insert 15 et de l'électrode 16 et un diamètre extérieur inférieur au diamètre de l'ouverture de l'extrémité tronconique 10 et supérieur à celui de la partie cylindrique 14, de manière à augmenter sensiblement le diamètre de passage à l'intérieur du manchon 3 grâce à l'insertion de l'écarteur 11 dans le manchon 3, et permettre le coulissement libre avec jeu du cathéter 4 dans l'écarteur 11.

Après cette insertion de l'écarteur 11 dans le manchon 3, on retire par un effort de traction modéré dans le sens de la flèche B le cathéter 4 comprenant la gaine souple 13, l'insert 15 et l'électrode 16.

Lors du retrait du cathéter 4, l'effort à fournir correspond au désengagement relatif du manchon 3 et de la gaine 4 ou au passage de l'électrode 16 sous les bossages en relief 19. L'écarteur 11 peut s'appuyer sur le manchon 3 au niveau de l'anneau 9, comme représenté Fig. 3, de sorte que l'effort à fournir pour désolidariser le manchon 3 du cathéter 4 ne met en cause que l'écarteur et la sonde, sans qu'il en résulte de traction ou de poussée sur la paroi cardiaque 1 ou la fibrine 2.

Le retrait du cathéter 4 s'effectue sans endommager notablement la fibrine 2 ou la paroi 1 du myocarde, en laissant en place le manchon 3 dans la fibrine 2.

Les étapes correspondantes sont avantageusement contrôlées par observation radioscopique des déplacements relatifs des parties radio-opaques 9, 15, 21.

En référence à la figure 3, après avoir effectué le retrait du cathéter, on peut intervenir directement sur la paroi 1 du myocarde pour observer , opérer des prélèvements à des fins d'analyse, effectuer une opération chirurgicale, ou désinfecter ladite paroi 1 par adjonction d'antibiotiques ou autres médicaments de contact.

Avantageusement, grâce à l'invention, on peut également remplacer le cathéter 4 par un cathéter neuf, sans endommager la paroi 1 du myocarde ou la fibrine 2 du fait que le manchon 3 déformable élastiquement reste en place pendant cette opération. Après insertion du cathéter neuf 4, l'écarteur 11 est retiré. Le manchon 3 reprend sa forme initiale par retour élastique, et se solidarise au cathéter 4.

## Revendications

1. Sonde cardiaque extractible au moyen d'un outil (11), notamment pour stimulateur cardiaque, du type comportant: un cathéter à gaine souple (4) portant une électrode (16) a son extrémité distale destinée à être placée contre une paroi interne du coeur; et un manchon (3) sensiblement cylindrique portant des moyens (5a, 5b) de fixation de la sonde sur la paroi cardiaque (1), apte à être solidarisé au cathéter (4) ; ledit cathéter (4) pouvant être désolidarisé dudit manchon (3) par action dudit outil (11) pour autoriser le retrait du cathéter (4) et le maintien en place du manchon (3) sur la paroi cardiaque ; le manchon (3) et le cathéter (4) comportant des formes coopérantes pour assurer leur solidarisation, caractérisée en ce que :
- le manchon (3) comporte, du côté tourné vers l'extrémité proximale de la sonde, une partie sensiblement tronconique (10) adaptée à recevoir l'outil d'écartement tubulaire (11) et,
- le manchon (3) est au moins partiellement déformable par agrandissement du diamètre de son passage intérieur au moyen de l'outil (11) d'écartement tubulaire, pour permettre l'extraction du cathéter par coulissement.

2. Sonde selon la revendication 1, caractérisée en ce que les formes coopérantes (17-20) du manchon (3) et du cathéter (4) sont des gorges coopérant avec des anneaux de retenue ou des bossages coopérant avec des cavités.

3. Sonde selon l'une quelconque des revendications 1 et 2, caractérisée en ce que le manchon (3) comporte à son extrémité distale une pièce (9) sensiblement indéformable.

4. Sonde selon la revendication 3, caractérisée en ce que ladite pièce (9) indéformable est un anneau de retenue en matériau radio-opaque.

5. Sonde selon la revendication 1, caractérisée en ce que le cathéter comporte, au voisinage de son extrémité distale, un insert (15) de retenue fixé sur la gaine souple (13).

6. Sonde selon la revendication 5, caractérisée en ce que ledit insert (15) est un tube en matériau radio-opaque.

## Claims

1. A cardiac probe extractable by means of an implement (11), in particular for cardiac stimulation, of the type comprising : a catheter (4) having a flexible sheath and at its distal tip an electrode (16) adapted to be placed against an inner surface of the heart; and an substantially cylindrical sleeve (3) having means (5a, 5b) for attachment of the probe to the cardiac surface (1), capable of being joined to the catheter (4) ; said catheter (4) being able to be disunited from said sleeve (3) by the action of said implement (11) to permit the withdrawal of the catheter (4) and the retention in position of the sleeve (3) on the cardiac surface ; the sleeve (3) and the catheter (4) comprising interacting shapes so that the sleeve and catheter lockingly engage one another, characterised in that :
- the sleeve (3) comprises, on its side turned toward the proximal tip of the catheter, a substantially frustoconical section (10) adapted to receive the tubular separation implement (11) and,
- the sleeve (3) is at least partially deformable through enlargement of the diameter of its inside through-pass by means of the tubular separation implement (11), in order to permit extraction of the catheter by sliding.

2. The probe as claimed in claim 1, characterised in that the interacting shapes (17-20) of the sleeve (3) and of the catheter (4) are grooves interacting with retention rings or bosses interacting with recesses.

3. The probe as claimed in claim 1 or 2, characterised in that the sleeve (3) comprises at its distal tip a substantially non-deformable component (9).

4. The probe as claimed in claim 3, characterised in that said non-deformable component (9) is a retention ring of radio-opaque material.

5. The probe as claimed in claim 1, characterised in that the catheter comprises, in the vicinity of its distal tip, a retention insert (15) attached to the flexible sheath (13).

6. The probe as claimed in claim 5, characterised in that said retention insert (15) is a tube of radio-opaque material.

## Patentansprüche

1. Mittels eines Werkzeugs (11) herausziehbare Herzsonde, insbesondere für einen Herzschrittmacher, von einer Bauart, die
einen Katheter mit biegsamer Hülle (4), der an seinem distalen Ende eine Elektrode (16) trägt, die dazu bestimmt ist, an einer inneren Herzwand angeordnet zu werden, und
eine im wesentlichen zylindrische Muffe (3) umfaßt, die Mittel (5a,5b) zur Befestigung der Sonde an der Herzwand (1) trägt und mit dem Katheter (4) fest verbunden werden kann, wobei der Katheter (4) von der Muffe (3) durch Betätigung des Werkzeugs (11) getrennt werden kann, um den Rückzug des Katheters (4) zuzulassen und die Muffe (3) an der Herzwand an ihrem Platz zu halten, wobei die Muffe (3) und der Katheter (4) zusammenwirkende Formen aufweisen, um ihre feste Verbindung zu sichern, dadurch gekennzeichnet, daß
- die Muffe (3) an ihrer dem proximalen Ende der Sonde zugewandten Seite einen im wesentlichen kegelstumpfförmigen Abschnitt (10) aufweist, der geeignet ist, das röhrenförmige Trennwerkzeug (11) aufzunehmen, und
- die Muffe (3) durch Vergrößerung ihres Innendurchmessers mittels des röhrenförmigen Trennwerkzeuges (11) zumindest teilweise verformbar ist, um das Herausziehen des Katheters durch Gleiten zu ermöglichen.

2. Sonde gemäß Anspruch 1, dadurch gekennzeichnet, daß die zusammenwirkenden Formen (17-20) der Muffe (3) und des Katheters (4) entweder Nuten sind, die mit Halteringen zusammenwirken, oder Vorsprünge, die mit Aussparungen zusammenwirken.

3. Sonde gemäß einem der Ansprüche 1 und 2, dadurch gekennzeichnet, daß die Muffe (3) an ihrem distalen Ende ein im wesentlichen unverformbares Teil (9) aufweist.

4. Sonde gemäß Anspruch 3, dadurch gekennzeichnet, daß das unverformbare Teil (9) ein Haltering aus strahlungsundurchlässigem Material ist.

5. Sonde gemäß Anspruch 1, dadurch gekennzeichnet, daß der Katheter nahe seinem distalen Ende einen an der biegsamen Hülle (13) befestigten Sicherungs-Einsatz (15) aufweist.

6. Sonde gemäß Anspruch 5, dadurch gekennzeichnet, daß der Einsatz (15) ein Rohr aus strahlungsundurchlässigem Material ist.
